**Europäisches Patentamt**

**European Patent Office**

**Office européen des brevets**

(19)

(11) Veröffentlichungsnummer: **0 392 497**

**A1**

(12) # EUROPÄISCHE PATENTANMELDUNG

(21) Anmeldenummer: **90106937.7**

(22) Anmeldetag: **11.04.90**

(51) Int. Cl.⁵: **A61K 7/06, A61K 33/00**

(30) Priorität: **11.04.89 DE 3911805**

(43) Veröffentlichungstag der Anmeldung:
**17.10.90 Patentblatt 90/42**

(84) Benannte Vertragsstaaten:
**AT BE CH DE DK ES FR GB GR IT LI LU NL SE**

(71) Anmelder: **Krautwurst, Gustav**
**Falkenweg 11**
**D-5485 Sinzig 1(DE)**

(72) Erfinder: **Krautwurst, Gustav**
**Falkenweg 11**
**D-5485 Sinzig 1(DE)**

(74) Vertreter: **von Füner, Alexander, Dr. et al**
**Patentanwälte v. Füner, Ebbinghaus, Finck**
**Mariahilfplatz 2 & 3**
**D-8000 München 90(DE)**

(54) **Haarbehandlungsmittel.**

(57) Verwendung von Lavalit nach Vermahlung auf Korngrößen bis 5 μm und darunter, wobei der Fraktionsanteil des Mahlguts mit einer Korngröße von 1 μm und weniger 40 Gew.-% ausmacht, zur Herstellung von Mitteln zur Förderung des Haarwuchses und Vermeidung bzw. Verlangsamung des Ergrauens von Haaren.

EP 0 392 497 A1

## HAARBEHANDLUNGSMITTEL

Aus "Hagers Handbuch der pharmazeutischen Praxis", 4. Ausgabe, Band 7B, Springer-Verlag, Berlin, 1977, Seite 269-283, ist die Anwendung verschiedener Silikate für pharmazeutische Zwecke bekannt. So werden Alumosilikate als Molekularsiebe vorgeschlagen, Perlite als Filterhilfsmittel und Tonmineralien, z.B. Kaolin, für pharmazeutisch-kosmetische Zwecke, z.B. bei der Herstellung vom Make-up-Präparaten und Akne-Lotionen. Kaolin wird auch vorgeschlagen wegen seiner Adsorptionsfähigkeit bei Behandlung des gastrointestinalen Trakts, mit anderen Worten als Adsorptionsmittel. Attapulgite werden ebenfalls im pharmazeutisch-chemischen Bereich vorgeschlagen, bei Anwendung ähnlich wie Kaolin, und zwar als Adsorptionsmittel. Bentonite sollen als Geliermittel für wässerige Systeme in der pharmazeutischen Technologie Anwendung finden. Über irgendwelche Wirkungen als Aktivsubstanz findet sich in dieser Literaturstelle nichts, im Gegenteil - alle diese Mittel werden gerade für die verschiedenen Zwecke vorgeschlagen, weil sie inert sind.

Demgegenüber wurde erfindungsgemäß festgestellt, daß Lavalit (Vulkangestein von Abraumhalden und aus dem Schlotbereich von erkalteten Vulkanen, z.B. in der Eifel (BRD)) nach Vermahlung auf Korngrößen bis 5 μm und darunter, wobei der Fraktionsanteil des Mahlgutes mit einer Korngröße von 1 μm und weniger 40 Gew.-% ausmacht, sehr gut zur Herstellung von Mitteln zur Förderung des Haarwuchses und zur Vermeidung und Bremsung des Ergrauens von Haaren dient.

Bei der Einnahme eines derartigen Mittels wurde außerdem auch festgestellt, daß durch die Absorptionsfähigkeit nicht nur unerwünschte Stoffe aus dem Magen- und Darmtrakt entfernt werden, sondern damit auch in gewissen Fallen eine Potenzsteigerung einhergeht.

Bei der Einnahme der Mittel wird der Ergrauungsprozeß der Haare gebremst, außerdem die einzelnen Haare verfestigt. Teilweise kommt es sogar zu neuem Haarwuchs.

Es wurden 10 Personen, 5 weibliche und 5 männliche, alle über 50 Jahre, getestet. Nach mehrwöchiger Einnahme wurde einheitlich festgestellt, daß bereits ergraute Haare nachdunkeln, der Haarwuchs an sich deutlich kräftiger wird und das einzelne Haar an Festigkeit gewinnt. Das Nachdunkeln der Haare steht in unmittelbarem Zusammenhang mit dem Haarwuchs an sich, so daß man annehmen kann, daß aus dem Mineral eine Substanz in das Haar eingelagert wird, die es nachdunkeln läßt. Es ist festgestellt worden, daß nicht nur eine Wirkung auf das Kopfhaar, sondern auch auf die Körperbehaarung zu beobachten ist, und gerade dort in verstärktem Maße, selbst in Bereichen, wo bereits trophische Störungen vorliegen. Auch das Nägelwachstum wird gefördert. Vier der 5 männlichen Patienten berichteten über eine Potenzsteigerung.

## Ansprüche

Verwendung von Lavalit nach Vermahlung auf Korngrößen bis 5μm und darunter, wobei der Fraktionsanteil des Mahlguts mit einer Korngröße von 1 und weniger 40 Gew.-% ausmacht, zur Herstellung von Mitteln zur Förderung des Haarwuchses und Vermeidung bzw. Verlangsamung des Ergrauens von Haaren.

## DOCUMENTS CONSIDERED TO BE RELEVANT

EP 90106937.7

| Category | Citation of document with indication, where appropriate, of relevant passages | Relevant to claim | CLASSIFICATION OF THE APPLICATION (Int Cl⁵) |
|---|---|---|---|
| X | <u>DE - A1 - 3 301 575</u><br>(F. ARIK et al.)<br>   * Zusammenfassung, Seite 2,<br>    Zeilen 28-33; Seite 3,<br>    Zeile 32 - Seite 4,<br>    Zeile 2 * | 1 | A 61 K 7/06<br>A 61 K 33/00 |
| X | <u>EP - A2 - 0 051 789</u><br>(R. BÄHR)<br>   * Zusammenfassung; Beispiel<br>    4; Anspruch 3 * | 1 | |
| A | <u>US - A - 3 935 129</u><br>(W.J. JABALEE)<br>   * Beispiel 1,5 * | 1 | |
| A | <u>US - A - 4 000 317</u><br>(W.C. MENDA et al.)<br>   * Patentansprüche * | 1 | |
| A | HANDBUCH DER KOSMETIKA UND RIECHSTOFFE, 2. Auflage, Band III, 1973, Dr. Alfred Hüthig Verlag, Heidelberg H. JANISTYN "Die Körperpflegemittel", Seiten 279,280<br>   * Seiten 279,280 * | 1 | **TECHNICAL FIELDS SEARCHED (Int Cl⁵)**<br><br>A 61 K 7/00 |

The present search report has been drawn up for all claims

| Place of search | Date of completion of the search | Examiner |
|---|---|---|
| WIEN | 27-06-1990 | IRMLER |